# EUROPEAN PATENT SPECIFICATION

(11) **EP 3 678 556 B1**
(45) Date of publication and mention of the grant of the patent: **09.10.2024**
(21) Application number: 18789233.6
(22) Date of filing: 06.09.2018
(51) Int. Cl.: A61B 8/08, A61B 8/00, A61B 1/04, A61B 1/00

(54) **IMAGING DEVICE**
BILDGEBUNGSVORRICHTUNG
DISPOSITIF D'IMAGERIE

(30) Priority: 06.09.2017 NL 2019493
(43) Date of publication of application: 15.07.2020
(73) Proprietor: De Raadt Beheer B.V., 2809 PB Gouda (NL); Van Seventer, Robert, 1058 JJ Amsterdam (NL)
(72) Inventor: VAN SEVENTER, Robert, 1058 JJ Amsterdam (NL); DE RAADT, Floris, 2809 PB Gouda (NL)
(74) Representative: EP&C
(86) International application number: PCT/NL2018/050574
(87) International publication number: WO 2019/050400

(56) References cited:
- US-A- 5 377 683
- US-A1- 2009 248 041
- US-A1- 2016 038 119

## Description

The present invention relates to an imaging device for use in medical interventions, and in particular in surgical procedures.

Ultrasound imaging systems are widely used to obtain images. Ultrasound typically uses sound waves with frequencies higher than the upper audible limit of human hearing to obtain images. This limit varies from person to person and is approximately 20 kilohertz in healthy, young adults. Ultrasound systems therefore operate with frequencies from 20 kHz up to several gigahertz.

In the medical field, ultrasound imaging systems are used to obtain ultrasound images of tissue, organs, nerves, arteries, veins, etc. Most ultrasound systems comprise a probe to be placed on the skin of a patients. By placement of the ultrasound probe, on the skin images of structures below the skin may be obtained. US 2016/038119 A1 discloses an imaging device for use in medical interventions comprising an ultrasound probe for acquiring an anatomical image of a human body and for locating a medical instrument with respect to the image

A drawback of the use of ultrasound imaging in medical procedures, is that, in some cases, the location of interest may be out of the field of view of the ultrasound probe. This may for example be caused by relatively deep locations/structures that cannot be reached by the field of view of the ultrasound probe or by structures that do not let through ultrasound waves, such as bone structures.

It is an aim of the invention to provide an imaging device for use in medical interventions, such as surgery, that facilitates improved imaging of relevant areas of the human body, for example in order to enter an epidural space of the human body, or at least to provide an alternative imaging device.

The present invention provides an imaging device for use in medical interventions, such as surgery, according to claim 1.

The ultrasound probe is configured to obtain first image data using ultrasound waves. The ultrasound probe is placed on the skin of a patient. The ultrasound probe comprises a transducer that emits ultrasound waves into the body of the patient and that receives reflected ultrasound waves from the body of the patient. The reflected ultrasound waves form first image data that can be used to construct an ultrasound image.

The ultrasound image may be used to determine a position of the cannula in the body of a patient.

The cannula is an elongate hollow body that can be introduced into a body of a patient, for example to introduce fluids into the body of the patient. The fluids may comprise medicaments for treatment of a patient. The cannula may also be used to introduce other objects into the patient, such as an RF needle that is used to ablate nerves or other tissue of the patient. The cannula may comprise a sharp distal point that allows the cannula to be pierced through the skin and into the body of the patient.

A direct vision camera, i.e. a camera that records image data within the frequency range of visible light, is arranged on or in the cannula to record visible light images seen from this location at or near a distal end of the cannula. The direct vision camera for example comprises a CMOS image sensor or CCD image sensor.

The direct vision camera is preferably a colour camera that provides real colours to identify anatomical structures and/or pathological conditions.

The imaging device of the present invention provides both an ultrasound image on the basis of first image data obtained with an ultrasound probe and a direct vision image on the basis of second image data obtained by a direct vision camera. The ultrasound image and the direct vision image may be displayed by a single display device, such that the ultrasound image and the direct vision image are visible for the user on the single display device. This allows the operator to quickly switch between viewing the ultrasound image and the direct vision image.

The provision of both an ultrasound image and a direct vision image, facilitates the easy and safe application of otherwise difficult interventional techniques under direct vision.

The ultrasound image and the direct vision image may be displayed next to each other, such that the operator of the imaging device can simultaneously see both the ultrasound image and the direct vision image. This allows the operator to quickly switch between the two images. In particular, when the ultrasound image provides insufficient information, the operator may look at the direct vision image, for example to verify the location of the cannula within the body of patient.

The imaging device may comprise a processing device to receive and process the first image data to provide the ultrasound image and/or the second image data to provide the direct vision image, wherein the display unit is connected to the processing device to receive and display the ultrasound image and/or the direct vision image. The processing device may for example be a PC, tablet or an embedded processor.

The imaging device, for example the processing device, may comprise a storage device to store the first image data and/or the second image data and/or any data resulting from processing the first image data and/or the second image data. The storage device may for example store the ultrasound images and/or the direct vision images. The first image data, as obtained by the ultrasound probe and the second image data, as recorded by the direct vision camera, may also be used to directly display an ultrasound image and a direct vision image on the display device and discarded, including processed data, thereafter. In an embodiment, the storage device may be, when desired, activated by the user to store relevant data for later use.

The imaging device comprises a selector to select a desired image mode from multiple image modes to display the ultrasound image and/or the direct vision image. The selector enables the operator of the imaging device to switch between two or more image modes, wherein different image modes may be optimized for different situations and/or applications. For example in a first image mode only the ultrasound image may be displayed, while in the second image mode only the direct vision image is displayed. The selector allows to switch between these first and second image modes. For example, the operator may select the first image mode to determine the position of the cannula on the basis of the ultrasound image. When this ultrasound image provides insufficient information, the operator may operate the selector to switch to the second image mode in which the direct vision image is displayed on the display device.

In an embodiment, the multiple image modes at least comprise a first image mode, in which the ultrasound image is large with respect to the direct vision image, and a second image mode, in which the direct vision image is large with respect to the ultrasound image. It has been found that displaying the, at that time, most desired image of the ultrasound image and the direct vision in a larger format, and simultaneously the less desired image in a smaller format, is convenient for the operator of the imaging device. Thus, when the ultrasound image is most useful, the ultrasound image is displayed relatively large with respect to the direct vision image. When desired, in particular when the ultrasound image provides insufficient information, the selector can be operated to switch to another image mode, in which the direct vision image is displayed relatively large with respect to the ultrasound image.

In an embodiment, the imaging device comprises a microphone device connected to the selector to select the desired image mode on the basis of vocal commands. The use of vocal commands to select the desired image mode has been found to be convenient for the operator of the imaging device. For example, the vocal command "ultrasound view" may be used to switch to an image mode in which the ultrasound image is prominently shown, and the vocal command "direct vision view" may be used to switch to an image mode in which the direct vision image is prominently shown.

In an embodiment, the direct vision camera is a fiberscope at least partially arranged into the longitudinal internal channel of the cannula. Fiberscopes are camera devices in which at least one optical fiber is used to propagate visible light. The advantage of these fiberscopes is that the optical fiber is relatively long and thin such that the optical fiber may be arranged into the cannula. The fiberscope may comprise a bundle of multiple optical fibers.

Typically, the fiberscope comprises at least one optical fiber having a first end and a second end, wherein a light source and a camera sensor are optically connected to the first end of the optical fiber. The light source emits light into the first end of the optical fiber such that the light travels through the optical fiber and leaves the optical fiber at its second end. This light may be reflected on different structures, such as tissue or bone, of the human body and received back by the second end of the optical fiber. The reflected light travels back through the optical fiber to the camera sensor that records the reflected light to obtain a direct vision image.

As the optical fiber can be placed in the cannula, the fiberscope allows the operator of the imaging device to obtain direct vision images from the distal tip of the cannula. The fiber scope may also be moved distally out of the cannula to record direct vision images from that point of view.

In an alternative embodiment, the direct vision camera may be mounted on the cannula. In this embodiment, the direct vision camera does not have to be separately manipulated with respect to the cannula to move the field of view of the direct vision camera to a desired viewpoint. Since the direct vision camera is mounted on the cannula, the field of view of the direct vision camera is fixed with respect to the cannula. Advantageously the field of view is directed to the area in front of the distal end of the cannula. The camera that is mounted on the cannula may for example comprise one or more optical fibers corresponding to the fiberscope but fixed on the cannula, or a camera sensor fixed on the cannula.

In an embodiment, the imaging device comprises an infusion pump unit, wherein the infusion pump unit is connected to the cannula to pump infusion fluid into the internal channel of the cannula. It may be advantageous to provide a fluid, such as a saline solution, into the cannula. This fluid may for example be used to flush the internal channel of the cannula to remove fatty tissue from the internal channel. The fluid may also be dispensed from the distal of the cannula to create a space in front of the distal end of the cannula in order to obtain a better view of this area.

The infusion pump unit may also be used to administer medicaments via the cannula into the body of the patient.

In an embodiment, the cannula comprises a Y connection piece comprising a first inlet port and a second inlet port. An infusion pump unit may be connected to one of the first inlet port and the second inlet port and a fiberscope may be guided into the cannula via the other of the first inlet port and the second inlet port.

In an embodiment, the first inlet port and/or the second inlet port comprises a one way valve that prevents fluid flow in proximal direction of the cannula. By providing a one way valve in the first inlet port and/or the second inlet port, it can be prevented that fluid leaks out of the proximal side of the cannula. This fluid may for example be bodily fluid from the patient and/or a fluid that is pumped into the cannula using an infusion pump unit.

The one-way valve or valves is/are preferably constructed such that a device, for example a fiber scope, RF needle or biopsy needle can be moved into and through the one-way valve in distal direction, and out of the one-way valve in proximal direction, preferably without the one-way valve losing its function of blocking fluid flow in the proximal direction. The one-way valve may for example be constructed as a flexible annular disc.

In an embodiment, a longitudinal axis of the first inlet port is coincident with a longitudinal axis of the cannula, wherein a longitudinal axis of the second inlet port is arranged at an angle of 20-70 degrees with the longitudinal axis of the cannula. It is advantageous that one of the first inlet port and the second inlet port has a longitudinal axis coincident with the longitudinal axis of the cannula, to allow the introduction of relatively stiff devices such as a RF needle or a biopsy needle. The other inlet port can be used for introduction of a fiberscope, or other more flexible devices, or the connection of an infusion pump unit.

In an embodiment, the cannula comprises a third inlet port. In an embodiment with a third inlet port, a surgical tool, such as for example a RF needle or a biopsy needle, and a fiberscope may at the same time be arranged in the cannula, and simultaneously an infusion pump unit may be connected to the cannula to introduce fluid into the cannula, without the need to use one inlet port for two or more of these devices. This allows a convenient operating of the cannula and the other devices.

The third inlet port may comprise a one-way valve that prevents fluid flow in proximal direction of the cannula.

The discloure also provides a method of positioning a cannula in a body of a patient and obtaining images from the body of the patient, using an imaging device as claimed in any of the claims 1-15, comprising the steps of:
inserting the cannula into the body of the patient,
monitoring a position of the cannula using the ultrasound image, and
when desired, viewing the direct vision image.

In an embodiment, the method comprises selecting, with a selector, a desired image mode from multiple image modes to display the ultrasound image and/or the direct vision image.

In an embodiment, viewing the direct vision image is performed to double check the position of the cannula and/or to identify anatomical structures and/or to identify pathological conditions of the body of the patient.

In an embodiment, the method comprises the step of positioning the direct vision camera in a desired position with respect to the cannula to obtain a desired viewpoint with the direct vision camera.

In an embodiment, the method comprises pumping infusion fluid into the internal channel of the cannula.

Embodiments of the invention will now be described, by way of example only, with reference to the accompanying schematic drawings in which corresponding reference symbols indicate corresponding parts, and in which:
Figure 1 shows an imaging device according to an embodiment of the invention;
Figure 2 shows a first image mode on the display device;
Figure 3 shows a second image mode on the display device;
Figure 4 shows a cannula according to a first embodiment of the invention;
Figure 5 shows a cannula according to a second embodiment of the invention; and
Figure 6 shows a cannula according to a third embodiment of the invention.

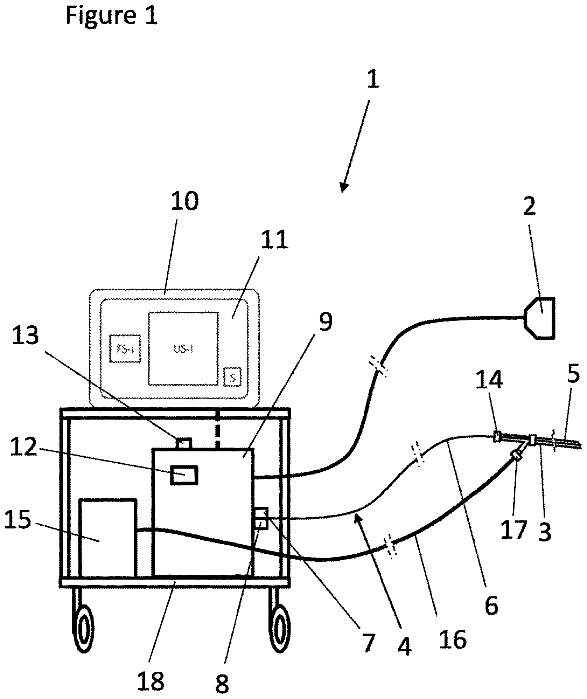

Figure 1 shows an imaging device according to an embodiment of the invention, generally denoted by reference numeral 1, for use in surgery, or other medical interventions. The imaging device 1 comprises an ultrasound probe 2 to obtain first image data on the basis of ultrasound waves transmitted and received by the ultrasound probe 2 while the ultrasound probe 2 is positioned on the skin of a patient. The ultrasound probe 2 comprises a transducer that emits ultrasound waves into the body of the patient and receives reflected ultrasound waves from the body of the patient. These reflected ultrasound waves form the first image data that can be used to construct an ultrasound image.

Further, the imaging device 1 comprises a cannula 3 and a fiberscope 4. The cannula 3 comprises an elongate body having a longitudinal internal channel 5. At its distal end, the cannula 3 a sharp distal point that allows the cannula 3 to be pierced through the skin and into the body of the patient

The fiberscope 4 comprises an elongate bundle of optical fibers 6 having a distal end and a proximal end. A distal part of the bundle of optical fibers 6 is arranged in the internal channel 5 of the cannula 3, whereby the distal end of the bundle of optical fibers 6 is arranged close to the distal end of the cannula 3. The fiberscope 4 enters the internal channel 5 of the cannula 3 at a first inlet port 14. At the proximal end of the bundle of optical fibers 6 a light source 7 and a camera sensor 8, for example a CMOS or CCD sensor, are arranged. The light source 7 and the camera sensor 8 are optically connected to the proximal end of the bundle of optical fibers. The light source 7 emits visible light, i.e. having a frequency in the spectrum of visible light, into the proximal end of the bundle of optical fibers 6.

This light propagates through the optical fibers 6 to the distal end of the bundle of optical fibers and out this distal end. This light illuminates a structure, such as tissue or bone, in front of the distal end of the bundle of optical fibers 6.

Reflected light will be received by the distal end of bundle of optical fibers and travel back to the proximal end of the bundle of optical fibers 6. One or more lenses may be provided at the distal end of the bundle of optical fibers 6 to focus the light on the distal end of the bundle of optical fibers 6. The reflected light will be received by the camera sensor 8 that is optically connected to the proximal end of the bundle of optical fibers 6. The camera sensor 8 records the reflected light 6 to obtain second image data that can be used to provide a direct vision image. Thus, in the position of the fiberscope 4 in Figure 1, the fiberscope 4 provides an image on the basis of visible light from the point of view of the distal end of the cannula 3 in distal direction of the cannula 3.

The fiberscope 4 can be moved with respect to the cannula 3. In particular, the bundle of optical fibers 6 may be moved further into and out of the internal channel 5 of the cannula 3 to change the point of view of the fiberscope 4 with respect to the cannula 3.

The ultrasound probe 2 and the fiberscope 4 are connected to a processing device 9 configured to process the first image data and the second image data. The processing device 9 is connected to a display device 10. The display device 10 comprises a display 11 to display an ultrasound image US-I on the basis of the first image data obtained by the ultrasound probe 2 and a direct vision image FS-I on the basis of the second image data obtained by the fiberscope 4. In the embodiment of Figure 1, the ultrasound image US-I and the direct vision image FS-I are displayed simultaneously on the same display 11. In other embodiments, the display 11 may selectively display one of the ultrasound image US-I and the direct vision image FS-I, in dependence of the instructions of the operator. In yet another embodiment, the ultrasound image US-I may be displayed on a first display and the direct vision image FS-I may be displayed on a second display, whereby the first display and the second display are arranged next to each other.

In dependence of the need of the operator of the imaging device 1, the operator may select between different image modes.

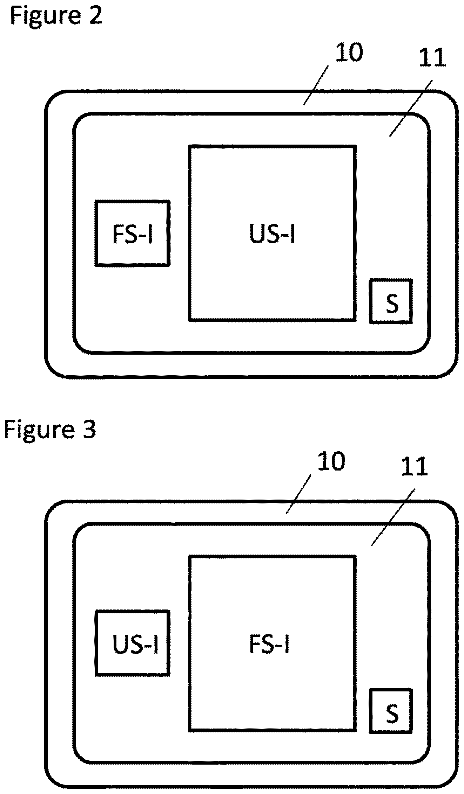

For example, in a first image mode as shown in Figure 2, the ultrasound image US-I is large with respect to the direct vision image FS-I. This image mode is advantageous when the operator will predominantly use the ultrasound image US-I to determine the position of the cannula with respect to the body of the patient. When the ultrasound image does no longer provide sufficient information, for instance when the cannula is arranged relatively deep into the patient and/or is arranged partially behind a bone structure that blocks ultrasound waves, or in order to double-check a position of the cannula 3, the operator may desire to predominantly use the direct vision image FS-I.

In such case the operator may select a second image mode as shown in Figure 3. In this second image mode of Figure 3, the direct vision image FS-I is large with respect to the ultrasound image US-I. This second image mode enables the operator to determine the position of the cannula mainly on the basis of the direct vision image FS-I, while advantageously the ultrasound image US-I is still displayed.

Thus, by switching between the first image mode and the second image mode the operator may see at the same location in his field of view on the display 10 either the ultrasound image US-I or the direct vision image FS-I.

As shown in Figure 1, the imaging device 1 comprises a selector 12 with which one of the image modes may be selected by the operator. To operate the selector 12, a selector button S may be provided on a touch-screen of the display 11. Alternatively a selector knob may be provided at any suitable location of the imaging device 1. Additionally or alternatively, the imaging device 1 comprises a microphone device 13 connected to the selector 12 to select the desired image mode on the basis of vocal commands. The use of vocal commands to select the desired image mode has been found to be convenient for the operator of the imaging device 1. For example, the vocal command "ultrasound view" may be used to switch to an image mode in which the ultrasound image US-I is prominently shown (Figure 2), and the vocal command "direction vision view" may be used to switch to an image mode in which the direct vision image FS-I is prominently shown (Figure 3).

It will be clear for the man skilled in the art that additional or alternative image modes are contemplated.

The imaging device 1 further comprises an infusion pump unit 15. The infusion pump unit 15 is configured to pump infusion fluid, in particular a saline solution to the cannula 3 via a fluid conduit 16. The fluid conduit 16 is connected to a second inlet port 17 of the cannula 3 so that fluid pumped by the infusion pump unit 15 will enter the internal channel 5 of the cannula 3 and run to the distal end of the cannula 3. This fluid may for example be used to flush the internal channel 5 of the cannula 3, in particular to remove fatty tissue from the internal channel 5. The fluid may also be dispensed from the distal of the cannula 3 to create a space in front of the distal end of the cannula 3 in order to obtain a better view with the fiberscope 4 on this area. Also, the infusion pump unit 15 may be used to introduce medicaments into the patient via the cannula 3.

The processing device 9, the display device 10 and the infusion pump unit 15 are arranged on a cart 18. When desired, the ultrasound probe 2, the cannula 3 and the fiberscope 4 may also be arranged on the cart 18 to easily displace the imaging device 1 to a desired location.

The selector 12 and the microphone device 13 are integrated parts of the processing device 9, but may also be separate devices. In Figure 1, the processing device 9 and the display device 10 are shown as separate devices. In practice, the processing device 9 and the display device 10 may also be integrated in a single device.

Further, it is remarked that the infusion pump unit may also be provided on a separate support, for example on an infusion pump support as known in the art.

The imaging device 1 according to the invention may be used in any suitable medical procedure. It may for example be used in epidural procedures, brain surgery, laparascopy, etc. The imaging device 1 may also be used for diagnostic medical interventions, for example to examine the state of tissue within the human body, such as the degree of inflammation of tissue.

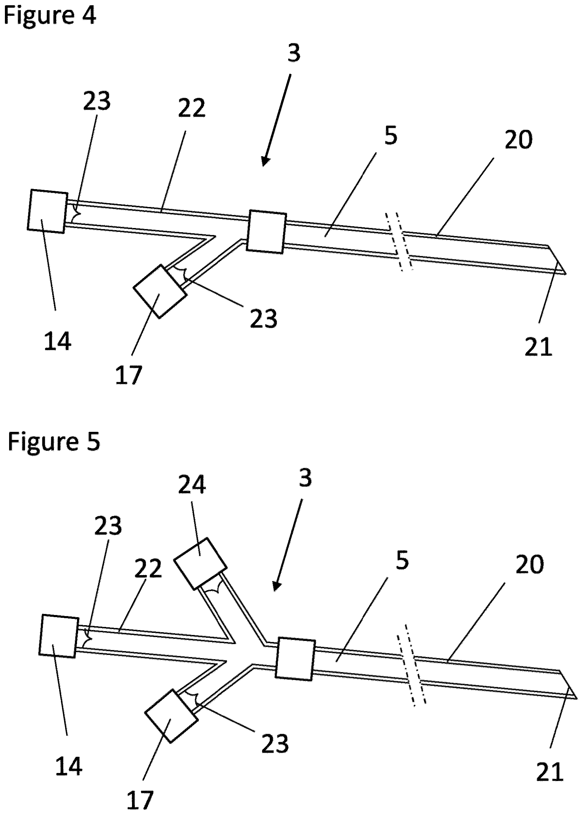

Figure 4 shows a cannula 3 according to an embodiment of the invention in more detail. The cannula comprises a elongate tube element 20 having at its distal end 21 a sharp distal point that allows the cannula 3 to be pierced through the skin and into the body of the patient. During use only a part of the tube element 20 will enter the body of a patient. At the proximal end of the cannula a Y-connection piece 22 is provided having the first inlet port 14 and the second inlet port 17.

The first inlet port 14 and the second inlet port 17 each comprise a one way valve 23 that prevents fluid flow in proximal direction of the cannula 3, i.e. from the internal channel 5 out of the first inlet port 14 or out of the second inlet port 17, respectively. The one-way valves 23 are constructed in such a way that they allow introduction of the fiberscope 4 and/or a surgical tool, such as an RF needle or biopsy needle, into the internal channel 5, while blocking, at the same time, a fluid flow from the internal channel in proximal direction of the cannula out of the first inlet port 14 or out of the second inlet port 17, respectively. In alternative embodiments, only one of the first outlet port 14 and the second outlet port 17 may be provided with such one-way valve 23. The one-way valves 23 shown in Figure 4 each comprise a flexible annular element that easily opens to allow fluid to flow to the distal end of the cannula 23, but blocks fluid flow in the opposite direction.

A longitudinal axis of the first inlet port 14 is coincident with a longitudinal axis of the cannula 3. This has the advantage that a relatively stiff device, such as a RF needle or a biopsy needle, can easily be introduced into the internal channel 5 of the cannula 3. A longitudinal axis of the second inlet port 17 may be arranged at an angle of for example 20-70 degrees with the longitudinal axis of the cannula. The second inlet port 17 can be used for introduction of a fiberscope 4 into the internal channel 5 of the cannula 3 or for the connection of the fluid conduit 16 of the infusion pump unit 15.

The outer diameter of the tube element may for example be in the range of 1.5-5 mm, for instance 1.8 mm or 2.4 mm and the inner diameter of the internal channel 5 may be in the range of 1 mm - 4.5 mm, for instance 1.6 mm or 1.7 mm. The length of the cannula 3 may be in the range of 5 mm - 25 mm, for instance 75 mm or 109 mm.

Figure 5 shows an alternative embodiment of a cannula 3. The cannula 3 of Figure 4 comprises a third inlet port 24. In the shown embodiment, the third inlet port 24 comprises a one-way valve 23 that prevents that prevents fluid flow from the internal channel 5 out of the third outlet port 23.

The embodiment the cannula 3 of Figure 5 is in particular suitable for simultaneous use of a surgical tool, such as for example a RF needle or a biopsy needle, and a fiberscope, while at the same time the cannula 3 is connected to an infusion pump unit.

For example, the first inlet port 14 may be used for introduction of a surgical tool into the internal channel 5 of the cannula, while the second inlet port 17 is used for introduction of a fiberscope 4 into the internal channel 5 of the cannula. The third inlet port 24 may be used for connection to an infusion pump unit 15 to pump infusion fluid into the internal channel 5 of eth cannula 3. The fiberscope 4 and the surgical tool may be arranged next to each other in the internal channel 5 without preventing or blocking longitudinal movement of the fiberscope 4 and the surgical tool with respect to each other or the cannula 3. Since the fiberscope 4 and the surgical tool enter the cannula at other inlet ports, the positions of the fiberscope 4 and the surgical tool can conveniently be operated.

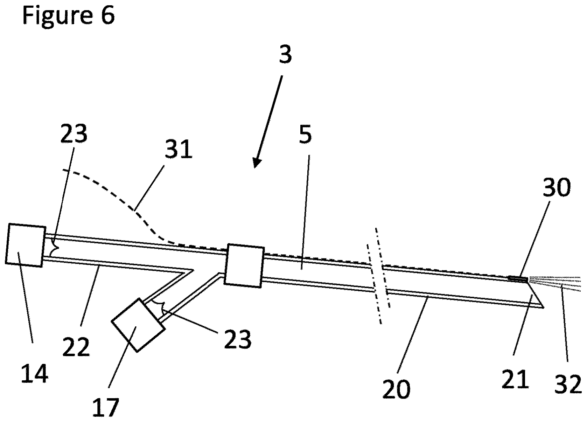

Figure 6 shows another alternative embodiment of a cannula 3 to be used in an imaging device according to the invention. In this embodiment a camera sensor 30 is mounted on the distal end of the this camera sensor 3 can be connected by a wire 31 to the processing device 9. The camera sensor is configured to record light, i.e. wave frequencies within the spectrum of visible light, The field of view 32 of the camera sensor 30 is directed to the area in front of the distal end of the cannula 3. This camera sensor 30 provides a good view on this area which may be advantageous when a surgical procedure is carried out with a surgical tool introduced into the body of the patient via the internal channel 5 of the cannula 3.

An advantage of the camera sensor 30 is that it does not have to be separately manipulated with respect to the cannula 3 to move the field of view of the camera sensor to a desired viewpoint. It will automatically move together with the movement of the cannula 3. However, this also means that there is less flexibility with respect to positioning the point of view of the camera sensor, when compared to a separate camera, such as a separate fiberscope.

In yet an alternative embodiment, a fiberscopic camera, a camera system using optical fibers, may be integrated in or on the cannula 3.

## Claims

1. An imaging device (1) for use in medical interventions, such as surgery, comprising:
an ultrasound probe (2) to provide first image data;
a cannula (3) having a longitudinal internal channel (5) and configured to be introduced into the human body;
a direct vision camera (4, 30) arranged, at least partially, on or in the cannula to
provide second image data; and
a display device (10) comprising at least one display (11) to display an ultrasound image (US-I) on the basis of the first image data and a direct vision image (FS-I) on the basis of the second image data, wherein the direct vision camera (4, 30) is a camera that records image data within the frequency range of visible light, wherein the imaging device comprises a selector (12) to select a desired image mode from multiple image modes to display the ultrasound image (US-I) and/or the direct vision image (FS-I).

2. The imaging device (1) of claim 1, wherein the multiple image modes at least comprise a first image mode, in which the ultrasound image (US-I) is large with respect to the direct vision image (FS-I), and a second image mode, in which the direct vision image is large with respect to the ultrasound image.

3. The imaging device (1) of claim 1 or 2, wherein the imaging device comprises a microphone device (13) connected to the selector (12) to select the desired image mode on the basis of vocal commands.

4. The imaging device (1) of any of the preceding claims, wherein the direct vision camera comprises a CCD sensor and/or CMOS sensor.

5. The imaging device (1) of any of the preceding claims, wherein the direct vision camera (4, 30) is a fiberscope at least partially arranged in the longitudinal internal channel (5) of the cannula (3).

6. The imaging device (1) of claim 5, wherein the fiberscope (4) comprises a light source (7), a camera sensor (8) and an optical fiber (6).

7. The imaging device (1) of any of the claims 1-4, wherein the direct vision camera (4, 30) is mounted on the cannula (3).

8. The imaging device (1) of any of the preceding claims, wherein the imaging device comprises an infusion pump unit (15), wherein the infusion pump unit is connected to the cannula (3) to pump infusion fluid into the internal channel (5) of the cannula.

9. The imaging device (1) of any of the preceding claims, wherein the cannula (3) comprises a Y connection piece (22) comprising a first inlet port (14) and a second inlet port (17).

10. The imaging device (1) of the preceding claim, wherein the first inlet port (14) and/or the second inlet port (17) comprises a one way valve (23) that prevents fluid flow in proximal direction of the cannula (3).

11. The imaging device (1) of claim 9 or 10, wherein a longitudinal axis of the first inlet port (14) is coincident with a longitudinal axis of the cannula (3), and wherein a longitudinal axis of the second inlet port (17) is arranged at an angle of 20-70 degrees with the longitudinal axis of the cannula.

12. The imaging device (1) of any of the claims 9-11, wherein the cannula (3) comprises a third inlet port (24).

13. The imaging device (1) of the preceding claim, wherein the third inlet port (24) comprises a one-way valve that prevents fluid flow in proximal direction of the cannula.

14. The imaging device (1) of claim 8 and any of the claims 9--13, wherein the infusion pump unit (15) is connected to one of the first inlet port (14) or the second inlet port (17) and a fiberscope (4) is guided into the cannula (3) via the other of the first inlet port or the second inlet port.

15. The imaging device (1) of any of the preceding claims, wherein the imaging device comprises a processing device (9) to receive and process the first image data to provide the ultrasound image (US-I) and/or the second image data to provide the direct vision image (FS-I), wherein the display device (10) is connected to the processing device to receive and display the ultrasound image and/or the direct vision image.

## Patentansprüche

1. Bildgebungsvorrichtung (1) zur Verwendung in medizinischen Eingriffen, wie etwa Chirurgie, umfassend:
eine Ultraschallsonde (2) zum Liefern erster Bilddaten;
eine Kanüle (3), die einen längserstreckten internen Kanal (5) aufweist und dafür ausgelegt ist, in den menschlichen Körper eingeführt zu werden;
eine Direktsichtkamera (4, 30), zumindest teilweise auf oder in der Kanüle angeordnet, um zweite Bilddaten zu liefern; und
eine Anzeigevorrichtung (10), umfassend mindestens eine Anzeige (11) zum Anzeigen eines Ultraschallbilds (US-I) auf der Grundlage der ersten Bilddaten und eines Direktsichtbilds (FS-I) auf der Grundlage der zweiten Bilddaten, wobei die Direktsichtkamera (4, 30) eine Kamera ist, die Bilddaten innerhalb des Frequenzbereichs von sichtbarem Licht aufzeichnet, wobei die Bildgebungsvorrichtung einen Selektor (12) zum Auswählen eines gewünschten Bildmodus aus mehreren Bildmodi zum Anzeigen des Ultraschallbilds (US-I) und/oder des Direktsichtbilds (FS-I) umfasst.

2. Bildgebungsvorrichtung (1) nach Anspruch 1, wobei die mehreren Bildmodi mindestens einen ersten Bildmodus, in dem das Ultraschallbild (US-I) in Bezug auf das Direktsichtbild (FS-I) groß ist, und einen zweiten Bildmodus, in dem das Direktsichtbild in Bezug auf das Ultraschallbild groß ist, umfassen.

3. Bildgebungsvorrichtung (1) nach Anspruch 1 oder 2, wobei die Bildgebungsvorrichtung eine Mikrofonvorrichtung (13) umfasst, verbunden mit dem Selektor (12), zum Auswählen des gewünschten Bildmodus auf der Grundlage von Sprachbefehlen.

4. Bildgebungsvorrichtung (1) nach einem der vorhergehenden Ansprüche, wobei die Direktsichtkamera einen CCD-Sensor und/oder einen CMOS-Sensor umfasst.

5. Bildgebungsvorrichtung (1) nach einem der vorhergehenden Ansprüche, wobei die Direktsichtkamera (4, 30) ein Faserendoskop ist, das zumindest teilweise in dem längserstreckten internen Kanal (5) der Kanüle (3) angeordnet ist.

6. Bildgebungsvorrichtung (1) nach Anspruch 5, wobei das Faserendoskop (4) eine Lichtquelle (7), einen Kamerasensor (8) und eine Lichtleitfaser (6) umfasst.

7. Bildgebungsvorrichtung (1) nach einem der Ansprüche 1-4, wobei die Direktsichtkamera (4, 30) auf der Kanüle (3) montiert ist.

8. Bildgebungsvorrichtung (1) nach einem der vorhergehenden Ansprüche, wobei die Bildgebungsvorrichtung eine Infusionspumpeneinheit (15) umfasst, wobei die Infusionspumpeneinheit mit der Kanüle (3) zum Pumpen von Infusionsfluid in den internen Kanal (5) der Kanüle verbunden ist.

9. Bildgebungsvorrichtung (1) nach einem der vorhergehenden Ansprüche, wobei die Kanüle (3) ein Y-Verbindungsstück (22) umfasst, das einen ersten Einlassport (14) und einen zweiten Einlassport (17) aufweist.

10. Bildgebungsvorrichtung (1) nach dem vorhergehenden Anspruch, wobei der erste Einlassport (14) und/oder der zweite Einlassport (17) ein Rückschlagventil (23) umfassen, das Fluidfluss in proximaler Richtung der Kanüle (3) verhindert.

11. Bildgebungsvorrichtung (1) nach Anspruch 9 oder 10, wobei eine Längsachse des ersten Einlassports (14) mit der Längsachse der Kanüle (3) zusammenfällt und wobei eine Längsachse des zweiten Einlassports (17) unter einem Winkel von 20 - 70 Grad mit der Längsachse der Kanüle angeordnet ist.

12. Bildgebungsvorrichtung (1) nach einem der Ansprüche 9-11, wobei die Kanüle (3) einen dritten Einlassport (24) umfasst.

13. Bildgebungsvorrichtung (1) nach dem vorhergehenden Anspruch, wobei der dritte Einlassport (24) ein Rückschlagventil umfasst, das Fluidfluss in proximaler Richtung der Kanüle verhindert.

14. Bildgebungsvorrichtung (1) nach Anspruch 8 und einem der Ansprüche 9-13, wobei die Infusionspumpeneinheit (15) mit dem ersten Einlassport (14) oder dem zweiten Einlassport (17) verbunden ist und ein Faserendoskop (4) über den anderen des ersten Einlassports oder des zweiten Einlassports in die Kanüle (3) geführt wird.

15. Bildgebungsvorrichtung (1) nach einem der vorhergehenden Ansprüche, wobei die Bildgebungsvorrichtung eine Verarbeitungsvorrichtung (9) umfasst zum Empfangen und Verarbeiten der ersten Bilddaten, um das Ultraschallbild (US-I) zu liefern, und/oder der zweiten Bilddaten, um das Direktsichtbild (FS-I) zu liefern, wobei die Anzeigevorrichtung (10) mit der Verarbeitungsvorrichtung verbunden ist zum Empfangen und Anzeigen des Ultraschallbilds und/oder des Direktsichtbilds.

## Revendications

1. Dispositif d'imagerie (1) destiné à être utilisé dans des interventions médicales, telles qu'une opération chirurgicale, comprenant :
une sonde échographique (2) destinée à fournir des données de première image ;
une canule (3) dotée d'un canal interne longitudinal (5) et configurée pour être introduite dans le corps humain ;
une caméra à vision directe (4, 30) agencée, au moins partiellement, sur ou dans la canule pour fournir des données de seconde image ; et
un dispositif d'affichage (10) comprenant au moins un afficheur (11) pour afficher une image échographique (US-I) sur la base des premières données d'image et une image de vision directe (FS-I) sur la base des secondes données d'image, dans lequel la caméra de vision directe (4, 30) est une caméra qui enregistre des données d'image dans la plage de fréquences de la lumière visible, dans lequel le dispositif d'imagerie comprend un sélecteur (12) pour sélectionner un mode d'image souhaité parmi des modes d'image multiples afin d'afficher l'image échographique (US-I) et/ou l'image de vision directe (FS-I).

2. Dispositif d'imagerie (1) selon la revendication 1, dans lequel les modes d'image multiples comprennent au moins un premier mode d'image, dans lequel l'image échographique (US-I) est grande par rapport à l'image de vision directe (FS-I), et un second mode d'image, dans lequel l'image de vision directe est grande par rapport à l'image échographique.

3. Dispositif d'imagerie (1) selon la revendication 1 ou 2, dans lequel le dispositif d'imagerie comprend un microphone (13) connecté au sélecteur (12) pour sélectionner le mode d'image souhaité sur la base de commandes vocales.

4. Dispositif d'imagerie (1) selon l'une quelconque des revendications précédentes, dans lequel la caméra de vision directe comprend un capteur CCD et/ou un capteur CMOS.

5. Dispositif d'imagerie (1) selon l'une quelconque des revendications précédentes, dans lequel la caméra de vision directe (4, 30) est un fibroscope au moins partiellement agencé dans le canal interne longitudinal (5) de la canule (3).

6. Dispositif d'imagerie (1) selon la revendication 5, dans lequel le fibroscope (4) comprend une source lumineuse (7), un capteur de caméra (8) et une fibre optique (6).

7. Dispositif d'imagerie (1) selon l'une quelconque des revendications 1 à 4, dans lequel la caméra à vision directe (4, 30) est montée sur la canule (3).

8. Dispositif d'imagerie (1) selon l'une quelconque des revendications précédentes, dans lequel le dispositif d'imagerie comprend une unité de pompe à perfusion (15), dans lequel l'unité de pompe à perfusion est raccordée à la canule (3) pour pomper le liquide de perfusion dans le canal interne (5) de la canule.

9. Dispositif d'imagerie (1) selon l'une quelconque des revendications précédentes, dans lequel la canule (3) comprend une pièce de raccordement en Y (22) comprenant un premier orifice d'entrée (14) et un deuxième orifice d'entrée (17).

10. Dispositif d'imagerie (1) selon la revendication précédente, dans lequel le premier orifice d'entrée (14) et/ou le second orifice d'entrée (17) comprennent une valve unidirectionnelle (23) qui empêche un écoulement de fluide dans la direction proximale de la canule (3).

11. Dispositif d'imagerie (1) selon la revendication 9 ou 10, dans lequel un axe longitudinal du premier orifice d'entrée (14) coïncide avec un axe longitudinal de la canule (3), et dans lequel un axe longitudinal du deuxième orifice d'entrée (17) est agencé selon un angle de 20 à 70 degrés avec l'axe longitudinal de la canule.

12. Dispositif d'imagerie (1) selon l'une quelconque des revendications 9 à 11, dans lequel la canule (3) comprend un troisième orifice d'entrée (24).

13. Dispositif d'imagerie (1) selon la revendication précédente, dans lequel le troisième orifice d'entrée (24) comprend une valve unidirectionnelle qui empêche un écoulement de fluide dans la direction proximale de la canule.

14. Dispositif d'imagerie (1) selon la revendication 8 et l'une quelconque des revendications 9 à 13, dans lequel l'unité de pompe à perfusion (15) est raccordée à l'un du premier orifice d'entrée (14) et du deuxième orifice d'entrée (17) et un fibroscope (4) est guidé dans la canule (3) par l'autre du premier orifice d'entrée et du deuxième orifice d'entrée.

15. Dispositif d'imagerie (1) selon l'une quelconque des revendications précédentes, dans lequel le dispositif d'imagerie comprend un dispositif de traitement (9) pour recevoir et traiter les premières données d'image afin de fournir l'image échographique (US-I) et/ou les secondes données d'image afin de fournir l'image de vision directe (FS-I), dans lequel le dispositif d'affichage (10) est connecté au dispositif de traitement pour recevoir et afficher l'image échographique et/ou l'image de vision directe.
